# EUROPEAN PATENT SPECIFICATION

(11) **EP 3 364 994 B1**
(45) Date of publication and mention of the grant of the patent: **13.12.2023**
(21) Application number: 16797662.0
(22) Date of filing: 24.10.2016
(51) Int. Cl.: A61K 38/01, A61K 31/198, A61K 31/20, A61K 31/7068, A61K 31/7072, A23L 33/00, A23L 33/12, A23L 33/13, A61K 31/122, A61K 31/14, A61K 31/202, A61K 31/355, A61K 31/375, A61K 31/455, A61K 31/59, A61K 33/04, A61K 38/02, A61P 25/00

(54) **METHOD FOR IMPROVING WHITE MATTER INTEGRITY IN HYPERPHENYLALANINEMIA AND PHENYLKETONURIA PATIENTS**
VERFAHREN ZUR VERBESSERUNG DER INTEGRITÄT VON WEISSER SUBSTANZ BEI PATIENTEN MIT HYPERPHENYLALANINÄMIE UND PHENYLKETONURIE
MÉTHODE PERMETTANT D'AMÉLIORER L'INTÉGRITÉ DE LA SUBSTANCE BLANCHE CHEZ DES PATIENTS ATTEINTS D'HYPERPHÉNYLALANINÉMIE ET DE PHÉNYLCÉTONURIE

(30) Priority: 23.10.2015 WO PCT/NL2015/050739
(43) Date of publication of application: 29.08.2018
(73) Proprietor: N.V. Nutricia, 2712 HM Zoetermeer (NL)
(72) Inventor: SAVELKOUL, Paul Johannes Maria, 3584 CT Utrecht (NL); VERKUIJL, Jan Maarten, 3584 CT Utrecht (NL); HAGEMAN, Robert Johan Joseph, 3584 CT Utrecht (NL); DE WILDE, Mattheus Cornelis, 3584 CT Utrecht (NL); COUNOTTE, Danielle Stefanie, 3584 CT Utrecht (NL); RAKHSHANDEHROO, Myryam, 3584 CT Utrecht (NL)
(74) Representative: Nederlandsch Octrooibureau
(86) International application number: PCT/NL2016/050733
(87) International publication number: WO 2017/069630

(56) References cited:
- WO-A1-2014/171813
- Laus M Broersen ET AL: "A specific multi-nutrient diet reduces Alzheimer-like pathology in young adult A[beta]PPswe/PS1dE9 mice", Journal of Alzheimer's disease : JAD, 1 January 2013 (2013-01-01), pages 177-190, XP055315661, Netherlands DOI: 10.3233/JAD-2012-112039 Retrieved from the Internet: URL:http://content.iospress.com/download/j ournal-of-alzheimers-disease/jad112039?id= journal-of-alzheimers-disease/jad112039
- BERTHOLD KOLETZKO ET AL: "Omega-3 LC-PUFA Supply and Neurological Outcomes in Children With Phenylketonuria (PKU)", JOURNAL OF PEDIATRIC GASTROENTEROLOGY AND NUTRITION, vol. 48, no. Suppl 1, 1 March 2009 (2009-03-01), pages S2-S7, XP055315223, US ISSN: 0277-2116, DOI: 10.1097/MPG.0b013e3181977399
- PETER J. ANDERSON ET AL: "White matter pathology in phenylketonuria*", MOLECULAR GENETICS AND METABOLISM, vol. 99, 1 January 2010 (2010-01-01), pages S3-S9, XP055315229, AMSTERDAM, NL ISSN: 1096-7192, DOI: 10.1016/j.ymgme.2009.10.005
- ANA M. ADAMO: "Nutritional factors and aging in demyelinating diseases", GENES & NUTRITION ; STUDYING THE RELATIONSHIP BETWEEN GENETICS AND NUTRITION IN THE IMPROVEMENT OF HUMAN HEALTH, vol. 9, no. 1, 6 December 2013 (2013-12-06), XP055315241, DE ISSN: 1555-8932, DOI: 10.1007/s12263-013-0360-8
- VIBEKE BRUINENBERG ET AL: "A Specific Nutrient Combination Attenuates the Reduced Expression of PSD-95 in the Proximal Dendrites of Hippocampal Cell Body Layers in a Mouse Model of Phenylketonuria", NUTRIENTS, vol. 8, no. 4, 26 March 2016 (2016-03-26), page 185, XP055315272, CH ISSN: 2072-6643, DOI: 10.3390/nu8040185

## Description

The invention is in the field of medical nutrition and more particularly relates to compositions for use in improving conditions associated with phenylketonuria (PKU) and hyperphenylalaninemia. In a preferred aspect, the invention pertains to improving myelin formation (myelination) and/or myelin protein and/or myelin lipid formation, and/or improving the function (preferably health and/or number) of oligodendrocytes, and/or improving axon integrity and quality, and/or improving white matter integrity in patients suffering from PKU or hyperphenylalaninemia. The invention particularly relates to white matter integrity in PKU conditions.

### Background

Phenylalanine is an essential amino acid, therefore it must be provided for in the diet, as it is vital for protein synthesis and normal growth and development. The first step in the metabolism of phenylalanine is the conversion of phenylalanine to tyrosine, a reaction catalyzed by phenylalanine hydroxylase that predominantly occurs within the liver. Tyrosine is then converted to the thyroid hormone thyroxine, the neurotransmitter dopamine, the adrenal hormones adrenaline and noradrenaline and the pigment melanin.

The genetic mutations characteristic for Phenylketonuria (PKU) impair the proper functioning of the enzyme phenylalanine hydroxylase, which is therefore unable to metabolize phenylalanine to tyrosine, and therefore phenylalanine accumulates within the body. Accumulated phenylalanine is converted by other pathways to various metabolites including phenyl pyruvic acid and phenyl acetic acid. These pathways and metabolites are found within healthy individuals, however in the PKU patient the absence of PAH results in these pathways occurring more frequently leading to abnormally high levels of the metabolites they generate. Increased concentrations of phenylalanine and its metabolites cause the disruption and inhibition of various biochemical processes, which is thought to lead to the clinical manifestations as seen in PKU.

PKU can greatly affect myelination, OPC and/or oligodendrocyte proliferation and axon quality, and white matter tracts in early stages of development. Both human and animal studies have shown a decrease in the myelination levels in the central nervous system where this reduction is caused by the transformation of oligodendrocytes to non-myelinating phenotypes. Research in PKU mice shows that in response to high phenylalanine [Phe], myelinating oligodendrocytes change into non-myelinating cells [Dyer et al. "Evidence for central nervous system glial cell plasticity in phenylketonuria". J Neuropathol Exp Neurol, 1996. 55(7): p. 795-814]. In addition, juvenile rats placed on a diet high in Phe show increased turnover of myelin components and inhibited myelin synthesis [Hommes et al. "Turnover of the fast components of myelin and myelin proteins in experimental hyperphenylalaninemia. Relevance to termination of dietary treatment in human phenylketonuria" J Inherit Metab Dis, 1982. 5(1): p. 21-7]. In early treated PKU, a large population of patients exhibits at least some white matter abnormalities and the severity of pathology is strongly associated with the control of the metabolic abnormality, as measured using plasma and brain phenylalanine levels. Specifically, the changes observed in white matter on MRI are elevated water content, intramyelinic edema, and immature or vacuolated myelin.

Yet the problem is not limited to the early development stages only. Using more advanced imaging methods, such as diffusion tensor imaging (DTI), it is possible to assess movement of water in white matter, which informs about the architecture of white matter fiber tracts. Most DTI studies in early treated PKU report a reduction in the apparent diffusion coefficient (ADC), which is explained by more water within the myelin or increased myelin turnover. Similarly, more recent studies confirm that reduced ADC levels are correlated with higher Phe levels in the preceding 12 months [Kono et al. "Diffusion-weighted MR imaging in patients with phenylketonuria: relationship between serum phenylalanine levels and ADC values in cerebral white matter. " Radiology, 2005. 236(2): p. 630-6]. These results confirm that white matter integrity is Phe-sensitive even in adulthood, thus suggesting that the white matter abnormalities are not solely due to Phe toxicity to developing oligodendrocytes or early steps of myelination but that even in adulthood white matter is sensitive to Phe-levels and/or other disease pathophysiological processes, providing a window of opportunity for treatment. This point is further substantiated by the fact that 6 months of tetrahydrobiopterin (BH(4)) treatment, which lowers Phe but only in PKU patients that are responsive to this therapy, improve white matter integrity [White, D.A., et al., White matter integrity and executive abilities following treatment with tetrahydrobiopterin (BH4) in individuals with phenylketonuria. Mol Genet Metab, 2013. 110(3): p. 213-7]. White matter integrity is thus believed to be linked to hyperphenylalaninemia and particularly the PKU pathophysiology not just in the early development phases but also in adulthood.

### SUMMARY OF THE INVENTION

White matter is a component of the central nervous system and consists mostly of glial cells (predominantly oligodendrocytes and astrocytes) and myelinated axons that transmit electrical signals from one brain region to another. Oligodendrocytes follow a program of proliferation, migration and differentiation during brain development and mature oligodendrocytes are able to form myelin. When oligodendrocytes encounter axons that are electrically active, these will wrap myelin around these axons, thereby insulating them, which increases the speed of transmission of signals. In a PKU patients, all of this can be compromised or impaired.

The inventors realized that in PKU patients, oligodendrocyte precursor cell ('OPC') and/or oligodendrocyte proliferation and differentiation into mature oligodendrocytes (OL) and/or myelin formation ('myelination') and/or axon integrity, and/or white matter integrity can be improved using a nutritional therapy aiming at reducing phenylalanine levels in the brain in combination with specific actives (i) stimulating myelin protein and/or myelin lipid formation and/or myelination, and/or (ii) promoting the health and number of oligodendrocytes and/or (iii) promoting axon integrity and quality, where all of (i) - (iii) are compromised or at risk of impairment in PKU and hyperphenylalaninemia patients. In contrast to neurodegenerative disorders, increases in phenylalanine in PKU and hyperphenylalaninemia are inherited diseases that develop from birth resulting in decreased cerebral protein synthesis, thereby potentially impairing the formation of synapses in the brain. The mechanism underlying the neurological problems in Alzheimer's disease and other dementias is therefore very different from the underlying mechanisms in PKU and hyperphenylalaninemia.

### DETAILED DESCRIPTION OF THE INVENTION

In one aspect, the invention pertains to a composition for use according to claim 1.

In another aspect, the invention pertains to a combination for use according to claim 8.

Myelination preferably involves myelin sheath formation in the central nervous system (CNS). In one embodiment, the invention particularly pertains to myelin quality in the CNS. In one embodiment, the 'treatment' is directed to promote OPC proliferation and maturation into OL, which is impaired or at risk of impairment in PKU patients. In one embodiment, the 'treatment' is directed to axon integrity. Associated therewith, treatment may involve the improvement, promotion, restoration or maintenance of white matter integrity, and/or reducing the risk of occurring of white matter integrity impairments such as associated with demyelination or myelin protein and/or lipid loss and/or OPC and/or proliferation and maturation deficits and/or axon integrity imbalances, characteristic to PKU patients. Treatment is directed to improvement, promotion, restoration or maintenance of white matter integrity, and/or reducing the risk of occurrence of white matter integrity impairments or white matter abnormalities.

Further details are provided here below.

In the above cases, the subject is preferably a human subject. The human subject suffering from PKU or hyperphenylalaninemia can be an infant or child up to 18 years of age, or an adult between 18 and 50 years of age.

The white matter abnormalities associated with PKU do not extend to white matter disease or periventricular white matter disease. White matter deficits in PKU patients are preferably associated with impairments in terms of myelination and/or OPC and/or oligodendrocyte proliferation and/or axon integrity. White matter deficits addressed in the invention are specific to PKU patients, and to be regarded distinct from impairment in white matter integrity associated with neurodegenerative disorders such as dementia. The specific neuronal and cognitive issues of PKU patients are linked to their genetic lack to metabolize phenylalanine to tyrosine.

In one embodiment of the invention, the use for preserving and improving cognitive function and reducing cognitive deficit in phenylketonuria or hyperphenylalaninemia patients and/or preserving and improving cognitive function and reducing cognitive deficit associated with phenylketonuria or hyperphenylalaninemia, particularly learning and memory, are excluded from the invention.

The composition and combination will be outlined in more detail here below.

Firstly, should the composition or combination of the invention involve the administration of proteinaceous matter, it should be `substantially devoid from Phe' or `essentially free of phenylalanine', meaning that less than 2 wt% phenylalanine, more preferably less than 1 wt% Phe, more preferably less than 0.5 wt% Phe, even more preferably less than 0.1 wt% Phe, most preferably less than 0.05 wt% Phe, based on the total proteinaceous content of the composition or combination, is present. Research in PKU mice shows that in response to high Phe, myelinating oligodendrocytes change into nonmyelinating cells.

In order to achieve such low Phe levels, a product according to the invention comprises proteinaceous matter comprising free amino acids (other than phenylalanine) and/or a non-allergenic protein source such as glycomacropeptide (GMP). According to the present invention, `free amino acids' are amino acids not coupled to other amino acids, but the term includes amino acids salts or di- and tripeptides such as cystine or gly-gly dipeptides. An absolute phenylalanine-free product can be obtained when using free amino acids, and the composition of the amino acids can be easily adapted to the nutritional requirements depending on the age of the patients. Such proteinaceous compositions tailored to PKU patients are available in the art. GMP is a protein originating from casein and is low in phenylalanine, and can improve the taste significantly without significantly increasing the phenylalanine content of the composition or combination. If the composition or combination comprises a protein fraction, it is preferred to involve a mixture of GMP supplemented with free amino acids to the extent desired and/or according to nutritional requirements. The composition or combination preferably comprises at least 50 wt%, preferably 70 - 90 wt% GMP based on the total protein content, preferably supplemented to 100% with free amino acids.

A preferred amino acid composition according to the present invention has a relatively high content of the branched chain amino acids (BCAA) leucine, isoleucine and valine. These amino acids can potentially block the transport of phenylalanine over the intestinal barrier and also over the blood-brain barrier thereby helping in lowering the levels of phenylalanine in the brain, but without wishing to be tied down to any theory, the inventors also believe that adding these amino acids would increase cerebral protein synthesis. The protein fraction preferably comprises at least 15 wt% of said branched chain amino acids (BCAA), more preferably between 15 and 35 wt%, even more preferably between 16 and 30 wt%, particularly 17 - 25 wt%, most preferably at least 18 wt% based on the total protein content.

The composition or combination of the invention preferably comprises L-leucine in free form, wherein the total amount of L-leucine provided by the proteinaceous matter amounts to at least 7 wt%, preferably at least 8 wt%, more preferably 9-20 wt%, most preferably 9 - 15 wt%, based on total proteinaceous matter. In the context of the invention, the terms 'leucine' and 'L-leucine' are used interchangeably.

In order to improve LAT1-mediated blood-brain barrier transport of large neutral amino acids (LNAAs), it is preferred that the sum of threonine, valine, histidine and methionine is at least 12 wt%, preferably at least 13 wt%, more preferably at least 14 wt%, even more preferably at least 15 - 60 wt%, more preferably 16 - 40 wt%, based on total proteinaceous matter (i.e. the sum of all amino acids, peptides and proteins and hydrolysates thereof).

In one embodiment, the composition or combination of the invention may comprise inositol and/or iodine, preferably at least inositol. In one embodiment, the composition or combination of the invention preferably comprises Fe in therapeutically effective amounts.

### Vitamin D, vitamin K

Without wishing to be tied down to any theory, the inventors believe that supplementation of high doses of vitamin D reduces demyelination and in addition enhances remyelination of axons. Vitamin D has a regulatory function in oligodendrocytes. The composition or combination of the invention comprises vitamins K and vitamin D, both in therapeutically effective amounts. Within the context of the present invention, `vitamin D' is understood to encompass vitamin D₂ (ergocalciferol), vitamin D₃ (cholecalciferol). Vitamin D is preferably present in the composition or combination of the invention in an amount to provide a daily dosage in the range of 1 to 100 µg, in particular in the range of 1 to 50 µg, preferably 2 - 25 µg, preferably at least 3 µg, more preferably at least 4 µg, even more preferably at least 5 µg, more in particular in the range of 5 to 25 µg, even more preferably at least 5 - 15 µg. In one embodiment, vitamin D is present in an amount in the range of 1 to 100 µg, in particular in the range of 2 to 50 µg, preferably 2 - 25 µg, preferably at least 3 µg, more preferably at least 4 µg, even more preferably at least 5 µg, more in particular in the range of 5 to 25 µg, even more preferably at least 5 - 15 µg per 100 ml of the (liquid) product. In the context of the invention, 1 IU of vitamin D is the biological equivalent of 0.025 µg. Hence, 1,000 IU is the biological equivalent of 25 µg.

Without wishing to be tied down to any theory, vitamin K is believed to inhibit oxidative cell death in primary cultures of OPCs. It suggests a role of vitamin K on OPC and/or oligodendrocyte proliferation and myelin formation. Vitamin K is also believed to be important for the biosynthesis of the myelin sphingolipid sulfatide. MK4, the predominant form of vitamin K, has the highest concentration in myelin-rich areas of the brain, thus rendering it plausible that vitamin K has a specific role in these brain regions. The combination or composition according to the invention comprises at least a therapeutically effective amount of vitamin K, including vitamin K2. Vitamin K₂, also known as "menatetrenone", "menaquinone-7", "menaquinone" or "menadione", is a group name for a family of related compounds, generally subdivided into short-chain menaquinones, with menatetrenone ("MK-4") as the most important member, and long-chain menaquinones, of which MK-7, MK-8 and MK-9 are nutritionally the most recognized. Within the context of the present invention, `vitamin K is understood to refer to vitamin K1, vitamin K2, menaquinone-4 (MK-4), menaquinone-7 (MK-7). It is preferred that in the composition, Vitamin K is present in an amount to provide a daily dosage in the range of 1 to 100 µg, in particular in the range of 5 to 50 µg, more in particular at least 7 µg, preferably at least 8 µg, more preferably at least 9 µg, even more preferably in the range of 10 to 50 µg, particularly at least 11, 12, 13, 14, 15 µg, preferably up to 40 µg, more preferably up to 30 µg. In one embodiment, vitamin K is present in an amount in the range 1 to 100 µg, in particular in the range of 5 to 50 µg, more in particular at least 7 µg, preferably at least 8 µg, more preferably at least 9 µg, even more preferably in the range of 10 to 50 µg, particularly at least 11, 12, 13, 14, 15 µg, preferably up to 40 µg, more preferably up to 30 µg per 100 ml of the (liquid) product. Preferably vitamin K is present as vitamin K1.

### DHA/EPA/DPA

The composition or combination of the invention comprises ω-3 long-chain polyunsaturated fatty acid (LC PUFA; having a chain length of 18 and more carbon atoms) selected from the group consisting of docosahexaenoic acid (22:6; DHA), eicosapentaenoic acid (20:5; EPA) and docosapentaenoic acid (22:5 ω-3; DPA), containing at least DHA, more preferably DHA and EPA. EPA is converted to DPA (ω-3), increasing subsequent conversion of DPA to DHA in the brain. Hence, the present composition or combination preferably also contains a significant amount of EPA, so to further stimulate *in vivo* DHA formation.

The LCPUFAs (DHA, EPA and/or DPA) are preferably provided as triglycerides, diglycerides, monoglycerides, free fatty acids or their salts or esters, phospholipids, lysophospholipids, glycerol ethers, lipoproteins, ceramides, glycolipids or combinations thereof. Preferably, the present composition or combination comprises at least DHA in triglyceride form. Suitable ω-3 LCPUFA and/or DHA sources include tuna oil, (other) fish oils, DHA-rich alkyl esters, algae oil, egg yolk, or phospholipids enriched with ω-3 LCPUFA e.g. phosphatidylserine-DHA. Preferably, a composition or combination according to the invention comprises fish oil providing the omega-3 LCPUFA(s). Another particularly suitable source for the omega-3 LCPUFA(s) is algae oil.

If EPA and/or DPA are present, the total daily dosage of DHA+EPA+DPA taken together is in the range of 0.25 to 5 g per day, more preferably 0.5 to 5 g per day, most preferably 1 to 2.5 g per day. In a preferred embodiment, these amounts are based on the total sum of DHA and EPA if present. DHA is preferably administered in an amount of at least 0.5 g per day, more preferably 0.5 to 2.5 g per day, most preferably at least 1 g per day.

In terms of the composition or combination, the proportion of ω-3 LCPUFA (more preferably DHA+EPA+DPA, most preferably DHA+EPA) of the total fatty acids in the composition is preferably 5 to 95 wt%, more preferably 10 to 80 wt%, most preferably 15 to 70 wt%, even more preferably 20 to 60 wt% of the total fatty acids. The present composition or combination preferably comprises 5 to 95 wt% DHA based on total fatty acids, preferably 10 to 75 wt% DHA based on total fatty acids, more preferably 10 to 60 wt%, even more preferably 10 - 50 wt%, more preferably 10-40 wt%, especially at least 20 wt% DHA, based on total fatty acids of the composition or combination. The present composition or combination preferably comprises 5 to 95 wt% EPA based on total fatty acids, preferably 5 to 75 wt% EPA, even more preferably 5 - 50 wt%, more preferably 5 - 25 wt%, most preferably 5 - 15 wt%, based on total fatty acids of the composition or combination. In terms of DHA content in a composition or combination in accordance with the present invention, the DHA content is preferably 0.5 - 1.5 g per 100 ml of the (liquid) product. The amount of EPA is preferably at least 0.1 g, more preferably at least 0.2 g of the product. The above-mentioned amounts take into account and optimize several aspects, including taste (e.g. too high LCP levels reduce taste, resulting in a reduced compliance).

In the combination or composition of the invention, the ratio of the weight of DHA to EPA is preferably larger than 1, more preferably 2:1 to 10:1, more preferably 2:1 to 5:1. The ratios take into account and optimize the balance between DHA and precursors thereof to ensure optimal effectiveness while maintaining low-volume formulations.

If arachidonic acid (AA) is present or administered, it concerns a very low amount of AA, expressed in terms of a DHA/AA weight ratio in the present composition or combination of at least 5, preferably at least 10, more preferably at least 15, preferably at least 20, most preferably at least 25. If AA is administered, it preferably amounts to less than 5 weight%, more preferably less than 2.5 weight.%, preferably less than 1 wt% based on total fatty acids of the composition or combination.

### Uridine, UMP

The combination and composition according to the invention comprise one or more of uridine, deoxyuridine, uridine phosphates, uracil, and acylated uridine. The combination and composition preferably comprises at least one uridine or an equivalent thereof selected from the group consisting of uridine (i.e. ribosyl uracil), deoxyuridine (deoxyribosyl uracil), uridine phosphates (UMP, dUMP, UDP, UTP), nucleobase uracil and acylated uridine. In one embodiment, cytidine, CMP, citicoline (CDP-choline) may also be applied in addition to uridine (equivalent).

Preferably, the combination and composition according to the invention comprise an uridine phosphate selected from the group consisting of uridine monophosphate (UMP), uridine diphosphate (UDP) and uridine triphosphate (UTP). In a preferred embodiment, the composition or combination comprises at least one of the aforementioned uridine phosphates. Most preferably the present composition or combination comprises UMP, as UMP is most efficiently being taken up by the body. Hence, inclusion of UMP in the present combination and composition enables a high effectivity or efficacy at the lowest dosage and/or the administration of a low volume to the subject. Preferably at least 50 weight% of the uridine in the present combination and composition is provided by UMP, more preferably at least 75 weight%, most preferably at least 95 weight%. Doses administered are given as UMP. The amount of uracil sources can be calculated taking the molar equivalent to the UMP amount (molecular weight 324 Dalton).

The present use preferably comprises the administration of uridine (the cumulative amount of uridine, deoxyuridine, uridine phosphates, nucleobase uracil and acylated uridine derivatives) in an amount of 0.05-5 g per day, preferably 0.1-2.5 g per day, more preferably 0.25-1 g per day. The present use preferably comprises the administration of a composition or combination comprising uridine in an amount of 0.05-5 g UMP per 100 ml liquid product, preferably 0.1-2.5 g UMP per 100 ml liquid product, more preferably 0.25-1 g per 100 ml liquid product. The terms product, composition and combination are used interchangeably. Preferably 1-40 mg UMP per kilogram body weight is administered per day, more preferably 5 - 35, even more preferably 5 - 30 mg UMP/kg body weight. The above amounts also account for any amounts of cytidine, cytidine phosphates and citicoline incorporated in the composition or combination.

### Choline

In a preferred embodiment, the combination and composition according to the present invention comprise choline, a choline salt and/or choline ester. Herein, the term 'choline' shall be considered to encompass all these equivalents. The choline salt is preferably selected from choline chloride, choline bitartrate, or choline stearate. The choline ester is preferably selected from the group consisting of phosphatidylcholine and lyso-phosphatidylcholine. The present use preferably comprises the administration of more than 0.05 g choline per day, preferably 0.1 to 2 g choline per day, more preferably 0.2 to 1 g choline per day, most preferably 0.2 to 0.5 g choline per day. The present composition or combination preferably comprises 0.05 to 2 g choline per 100 ml of the liquid product, preferably 0.2 to 1 g, more preferably up to 0.5 g choline per 100 ml. The above numbers are based on choline, the amounts of choline equivalents or sources can be calculated taking the molar equivalent to choline into account, based on the molar mass of 104 g choline / mol.

### Phospholipids

Like choline and PUFAs, phospholipids are key components in myelin-enriched lipids and precursors, and in PKU patients these are often present at lower levels. Preferably, the composition and combination according to the present invention comprises phospholipids, preferably 0.1-50 weight% phospholipids based on total weight of lipids, more preferably 0.5-20 weight%, more preferably between 1 and 10% weight%, most preferably between 1 and 5 weight% based on total weight of lipids. The present use preferably comprises the administration of 50-1000 mg phospholipids. The total amount of lipids is preferably between 10 and 30 weight% on dry matter, and/or between 2 and 10 g lipid per 100 ml for a liquid product. The composition or combination preferably comprises between 0.01 and 1 gram lecithin per 100 ml, more preferably between 0.05 and 0.5 gram lecithin per 100 ml. A composition with these preferred amounts was found to be very effective. In one embodiment, the amount of phospholipids is between 0.01 and 0.5 g, more preferably between 0.05 and 0.25 g per 100 ml.

### B vitamins

The combination and composition according to the present invention preferably comprise at least one B complex vitamin. The vitamin B is selected from the group of vitamin B1 (thiamine), vitamin B2 (riboflavin), vitamin B3 (niacin or niacinamide), vitamin B5 (panthotenic acid), vitamin B6 (pyridoxine, pyridoxal, or pyridoxamine, or pyridoxine hydrochloride), vitamin B7 (biotin), vitamin B9 (folic acid or folate), and vitamin B12 (various cobalamins).

Preferably at least one, more preferably at least two B vitamins are selected from the group consisting of vitamin B6, vitamin B12 and vitamin B9, preferably vitamins B6 and/or B 12.

The vitamin B is to be administered in an effective dose, which dose depends on the type of vitamin B used. As a rule of thumb, a suitable minimum or a maximum dose may be chosen based on known dietary recommendations, for instance as recommended by Institute of Medicine (IOM) of the U.S. National Academy of Sciences or by Scientific Committee on Food (a scientific committee of the EU), the information disclosed herein and optionally a limited amount of routine testing. A minimum dose may be based on the estimated average requirement (EAR), although a lower dose may already be effective. A maximum dose preferably does not exceed the tolerable upper intake levels (UL), as recommended by IOM.

If present or administered, the vitamin B6 is preferably present in an amount to provide a daily dosage in the range of 0.1 to 50 mg, in particular in the range of 0.5 to 10 mg, more in particular in the range of 0.5 to 5 mg. The present composition or combination preferably comprises 0.1 to 50 mg vitamin B6 per 100 ml (liquid) product, more preferably 0.5 to 10 mg vitamin B6 per 100 ml (liquid) product, more preferably 0.5 to 5 mg vitamin B6 per 100 ml (liquid) product.

If present or administered, the vitamin B12 is preferably present in an amount to provide a daily dosage in the range of 0.5 to 15 µg, in particular in the range of 1 to 10 µg, more in particular in the range of 1 to 5 µg. The present composition or combination preferably comprises 0.5-15 µg vitamin B12 per 100 ml (liquid) product, more preferably 1 to 10 µg vitamin B12 per 100 ml (liquid) product, more preferably 1 to 5 µg vitamin B12 per 100 ml (liquid) product.

Throughout the application, the terms `folic acid', `folate' and 'B9' are used interchangeably. If present or administered, the vitamin B9 is preferably present in an amount to provide a daily dosage in the range of 0.05 to 1 mg, in particular in the range of 0.05 to 0.5 mg, preferably up to 0.4 mg, more preferably up to 0.3 mg, even more in particular in the range of 0.05 to 0.2 mg, preferably below 0.19 mg, below 0.18 mg, below 0.17 mg, below 0.16 mg, particularly 0.05 - 0.15 mg, most preferably up to 0.1 mg per day. The present composition or combination preferably comprises 0.05 to 1 mg folic acid per 100 g (liquid) product, more preferably 0.05 to 0.5 mg folic acid per 100 ml (liquid) product, preferably up to 0.4 mg, more preferably up to 0.3 mg, even more preferably 0.05 to 0.2 mg folic acid per 100 ml (liquid) product, preferably below 0.19 mg, below 0.18 mg, below 0.17 mg, below 0.16 mg, most preferably 0.05 - 0.15 mg, most preferably up to 0.1 mg folic acid per 100 ml product. Folates include folic acid, folinic acid, methylated, methenylated and formylated forms of folates, their salts or esters, as well as their derivatives with one or more glutamic acid, and all in either reduced or oxidized form.

If B vitamins are present, it is preferably vitamins B6 and/or B12.

### Vitamins C, E, selenium

The combination and composition of the invention preferably involves at least one, preferably at least two, most preferably all of the antioxidants selected from the group consisting of selenium, vitamin C and vitamin E. Selenium is the most preferred antioxidant. It plays a role in cholesterol levels, where cholesterol in an important myelin component.

Vitamin C may be present or administered in an amount to provide a daily dosage in the range of 0.01 to 2 g, in particular in the range of 0.025 to 0.5 g, more in particular in the range of 0.04 to 0.15 g. In one embodiment, vitamin C is given in an amount in the range of 0.025 to 2 g, in particular in the range of 0.04 to 0.5 g, more in particular in the range of 0.04 to 0.15 g per 100 ml of the (liquid) product.

Tocopherol (i.e. a compound having vitamin E activity) may be present in an amount to provide a daily dosage in the range of 0.01 to 0.5 g, in particular in the range of 0.01 to 0.25 g, more in particular in the range of 0.02 to 0.1 g, particularly 0.025 to 0.05 g, to prevent oxidative damage resulting from dietary PUFA. In one embodiment, tocopherol is present in an amount in the range of 0.01 to 0.5 g, in particular in the range of 0.01 to 0.25 g, more in particular in the range of 0.02 to 0.1 g, particularly 0.025 to 0.05 g per 100 ml of the product. The term "tocopherol", and 'alpha-TE', as used in this description, comprises tocopherols, tocotrienols, pharmaceutical and/or nutritional acceptable derivatives thereof and any combination thereof. The above numbers correspond to the amount of alpha-tocopherol, recognized in the art.

The present composition preferably contains selenium, because of its excellent antioxidant activity. The present use preferably provides the administration of a composition or a combination comprising between 0.01 and 5 mg selenium per 100 ml liquid product, preferably between 0.025 and 0.1 mg selenium per 100 ml liquid product. The amount of selenium administered per day is preferably more than 0.01 mg, more preferably 0.01 to 0.5 mg, most preferably at least 0.025 mg per day.

The combination or composition preferably further comprises L-leucine.

The combination or composition according to the above embodiments preferably comprise L-leucine, wherein the total amount of L-leucine provided by the proteinaceous matter amounts to at least 7 wt%, preferably at least 8 wt%, more preferably at least 9 - 20 wt%, more preferably 9 - 15 wt%, based on total proteinaceous matter. The proteinaceous matter is essentially free of phenylalanine. It is preferred that the sum of threonine, valine, histidine and methionine is at least 12 wt%, preferably at least 13 wt%, more preferably at least 14 wt%, preferably at least 15-60 wt%, more preferably 16 - 40 wt%, based on total proteinaceous matter. Further details about the proteinaceous matter are outlined here above.

The combination or composition of the invention according to the above embodiments preferably further comprises therapeutic amounts of choline and of at least one, preferably at least two of said B vitamins. These components and preferred amounts are outlined here above. These components and preferred amounts are outlined here above. With the above restraints, the invention preferably pertains to a composition or combination as defined here above, comprising administering, per daily dosage or per 100 ml product, at least 3, 4, 5, 6, 7, 8, 9 or all of:
- 0.25 to 5 g, preferably 0.5 to 5 g, more preferably 1 to 2.5 g of DHA+EPA+DPA taken together;
- 0.05-5 g, preferably 0.1-2.5 g, more preferably 0.25-1 g of uridine;
- 0.1 to 2 g, preferably 0.2 to 1 g, more preferably 0.2 to 0.5 g choline;
- 0.05 - 0.6 g, preferably 0.05 - 0.6 g, more preferably 0.06 - 0.2 g phospholipids;
- 0.1 to 50 mg, preferably 0.5 to 10 mg, more preferably 0.5 to 5 mg of vitamin B6;
- 0.5 to 15 µg, preferably 1 to 10 µg, more preferably 1 to 5 µg of vitamin B12;
- 0.05 to 0.5 mg, preferably 0.05 to 0.2 mg, preferably less than 0.19 mg, more preferably less than 0.18 mg, preferably less than 0.17 mg, more preferably less than 0.16 mg, more preferably 0.05 to 0.15 mg vitamin B9;
- 0.01 to 2 g, preferably 0.025 to 0.5 g, more preferably 0.04 to 0.15 g of Vitamin C;
- 0.01 to 0.5 g, preferably 0.015 to 0.25 g, more preferably 0.02 to 0.1 g of alpha-tocopherol; and
- more than 0.01 mg, preferably 0.01 to 0.5 mg, more preferably at least 0.025 mg selenium.

The composition or combination may further comprise, per daily dosage or per 100 ml product:
- 1 to 100 µg, in particular in the range of 1 to 50 µg, preferably 2 - 25 µg, preferably at least 3 µg, more preferably at least 4 µg, even more preferably at least 5 µg, more in particular in the range of 5 to 25 µg, even more preferably at least 5 - 15 µg of vitamin D; and
- 1 to 100 µg, preferably 5 to 50 µg, preferably at least 7 µg, preferably at least 8 µg, more preferably at least 9 µg, even more preferably in the range of 10 to 50 µg, particularly at least 11, 12, 13, 14, 15 µg, preferably up to 40 µg, more preferably up to 30 µg of Vitamin K, preferably vitamin K1.

Preferably L-leucine is also present in the aforementioned amounts and/or the sum of threonine, valine, histidine and methionine is preferably at least 12 wt%, preferably at least 13 wt%, more preferably at least 14 wt%, preferably at least 15 - 60 wt%, more preferably 16 - 40 wt%, based on total proteinaceous matter.

More preferably, the composition comprises, per daily dose or preferably per 100 ml composition:
0.1 - 0.5 g, preferably 0.2-0.4 g EPA,
0.5 - 1.5 g, preferably 0.75-1 g DHA,
0.1 - 0.5 g, preferably 0.2-0.4 g choline,
0.05 - 0.5 g, preferably 0.06-0.2 g phospholipids,
0.25 - 0.8 g, preferably 0.4 - 0.7 g UMP (uridine monophosphate),
0.01 - 0.05 g, preferably 0.015-0.04 g vitamin E (alpha-TE),
0.04 - 0.1 g, preferably 0.04-0.09 g vitamin C,
0.035 - 0.08 mg, preferably 0.035-0.07 mg selenium,
1 - 5 µg, preferably 2-4 µg vitamin B12,
0.5 - 3 mg, preferably 0.5-2 mg vitamin B6, and
0.05 - 0.2 mg, preferably less than 0.19 mg, more preferably less than 0.18 mg, preferably less than 0.17 mg, more preferably less than 0.16 mg, preferably 0.05-0.15 mg, more preferably 0.05 - 0.1 mg folic acid.

The composition further comprises vitamin D and vitamin K in the aforementioned amounts. Preferably L-leucine is also present in the aforementioned amounts and/or the sum of threonine, valine, histidine and methionine is preferably at least 12 wt%, preferably at least 13 wt%, more preferably at least 14 wt%, preferably at least 15-60 wt%, more preferably 16 - 40 wt%, based on total proteinaceous matter.

In one aspect of the invention, the compositions either as complete nutrition or as a supplement as detailed above are intended for use in the treatment of phenylketonuria patients.

### EXAMPLES

### Example 1 - Exemplary composition for use according to the invention

| | invention formula (per serving or per day) |
|---|---|
| Energy (kcal) | 173.5 |
| Protein/Protein Equivalent* (g) | 20 |
| | |
| Carbohydrates (g) | 16.5 |
| Fat (g) | 3.05 |
| - DHA (22:6n-3; mg) | 0.88 |
| Micronutrients* | |
| Calcium (mg) | 356 |
| Phosphorus (mg) | 276 |
| Vitamin E (mg α-TE) | 33.6 |
| Vitamin C (mg) | 44.4 |
| Vitamin D (µg) | 6 |
| Vitamin K (µg) | 20 |
| Folic acid (µg) | 160 |
| Niacin / Vitamin B3 (mg) | 7.1 |
| Vitamin B6 (mg) | 0.58 |
| Vitamin B12 (µg) | 1.8 |
| Choline (mg) | 153 |
| Nucleotides | |
| Uridine-5'-monophosphate (mg) | 625 |

| | |
|---|---|
| * The term 'protein equivalent' is an art-recognized term to express the amounts of the free amino acids as the amount of amino acids as if it was part of a protein, i.e. the weight value of amino acids is understood as the protein equivalent weight value, unless otherwise specified. The contribution of the amino acids to protein represents about 81% of the weight of the individual amino acids. | |

## Claims

1. A composition for use in therapeutically improving, promoting, restoring or maintaining white matter integrity in a subject suffering from PKU and/or in a subject suffering from or at increased risk of white matter abnormalities or impaired white matter integrity associated with PKU, said composition comprising therapeutic amounts of ω-3 polyunsaturated fatty acid (LCPUFA) selected from the group consisting of docosahexaenoic acid (DHA), eicosapentaenoic acid (EPA) and docosapentaenoic acid (DPA); and one of more of uridine, deoxyuridine, uridine phosphates, uracil, and acylated uridine, said composition comprising at least DHA, said composition further comprising therapeutic amounts of vitamin D and vitamin K, and wherein the composition further comprises proteinaceous matter which is essentially free from phenylalanine.

2. The composition for use according to claim 1, wherein the composition further comprises inositol and/or iodine.

3. The composition for use according to any of the preceding claims, wherein the composition comprises therapeutic amounts of at least one of B vitamins selected from the group consisting of vitamin B6, vitamin B12 and vitamin B9, preferably B6 and/or B12.

4. The composition for use according to any of the preceding claims, wherein the composition further comprises therapeutic amounts of choline, a choline salt and/or choline ester.

5. The composition for use according to any one of the preceding claims, wherein the composition further comprises therapeutic amounts of at least one, preferably at least two, most preferably all of the antioxidants selected from the group consisting of selenium, vitamin C and vitamin E, wherein the composition preferably comprises at least selenium.

6. The composition for use according to any of the preceding claims, wherein the sum of threonine, valine, histidine and methionine is at least 12 wt%, preferably at least 13 wt%, more preferably at least 14 wt%, more preferably 15 - 60 wt%, most preferably 16 - 40 wt% based on total proteinaceous matter.

7. The composition for use according to any of the preceding claims, wherein the composition comprises leucine in free form, wherein the total amount of L-leucine provided by the proteinaceous matter amounts to at least 7 wt%, preferably at least 8 wt%, more preferably at least 9 - 20 wt%, most preferably 9 - 15 wt%, based on total proteinaceous matter.

8. A combination of therapeutic amounts of ω-3 polyunsaturated fatty acid (LCPUFA) selected from the group consisting of docosahexaenoic acid (DHA), eicosapentaenoic acid (EPA) and docosapentaenoic acid (DPA); and one of more of uridine, deoxyuridine, uridine phosphates, uracil, and acylated uridine, for use in therapeutically improving, promoting, restoring or maintaining white matter integrity in a subject suffering from PKU and/or in a subject suffering from or at increased risk of white matter abnormalities or impaired white matter integrity associated with PKU, said combination comprising at least DHA and further comprising therapeutic amounts of vitamin D and vitamin K, and wherein the combination further comprises proteinaceous matter which is essentially free from phenylalanine.

9. A composition suitable for PKU patients, comprising therapeutic amounts of ω-3 polyunsaturated fatty acid (LCPUFA) selected from the group consisting of docosahexaenoic acid (DHA), eicosapentaenoic acid (EPA) and docosapentaenoic acid (DPA); one of more of uridine, deoxyuridine, uridine phosphates, uracil, and acylated uridine, and vitamin D and vitamin K, wherein the composition comprises at least DHA and further comprises proteinaceous matter which is essentially free from phenylalanine.

10. The composition according to claim 9, further comprising therapeutic amounts of at least one, preferably at least two, more preferably all of B vitamins selected from the group consisting of vitamin B6, vitamin B12 and vitamin B9.

11. The composition according to claim 9 or 10, wherein the sum of threonine, valine, histidine and methionine is at least 12 wt%, preferably at least 13 wt%, more preferably at least 14 wt%, more preferably 15 - 60 wt%, most preferably 16-40 wt% based on total proteinaceous matter and/or wherein the composition comprises leucine in free form, wherein the total amount of L-leucine provided by the proteinaceous matter amounts to at least 7 wt%, preferably at least 8 wt%, more preferably at least 9 - 20 wt%, most preferably 9 - 15 wt%, based on total proteinaceous matter.

## Patentansprüche

1. Zusammensetzung zur Verwendung bei der therapeutischen Verbesserung, Förderung, Wiederherstellung oder Aufrechterhaltung der Integrität der weißen Substanz bei einem Patienten, der an PKU leidet, und/oder bei einem Patienten, der an Anomalien der weißen Substanz oder einer beeinträchtigten Integrität der weißen Substanz im Zusammenhang mit PKU leidet oder ein erhöhtes Risiko dafür hat, wobei die besagte Zusammensetzung therapeutische Mengen einer mehrfach ungesättigten ω-3-Fettsäure (LCPUFA) umfasst, die aus der Gruppe ausgewählt ist, die aus Folgendem besteht: Docosahexaensäure (DHA), Eicosapentaensäure (EPA) und Docosapentaensäure (DPA); und mehreren von Uridin, Desoxyuridin, Uridinphosphaten, Uracil oder acyliertem Uridin, wobei die besagte Zusammensetzung mindestens DHA umfasst, wobei die besagte Zusammensetzung ferner therapeutische Mengen an Vitamin D und Vitamin K umfasst, und wobei die Zusammensetzung ferner eine proteinhaltige Substanz umfasst, die im Wesentlichen frei von Phenylalanin ist.

2. Zusammensetzung zur Verwendung nach Anspruch 1, wobei die Zusammensetzung ferner Inositol und/oder Jod umfasst.

3. Zusammensetzung zur Verwendung nach einem der vorhergehenden Ansprüche, wobei die Zusammensetzung therapeutische Mengen von mindestens einem der B-Vitamine umfasst, die aus der Gruppe ausgewählt sind, die aus Vitamin B6, Vitamin B12 und Vitamin B9, vorzugsweise B6 und/oder B12, besteht.

4. Zusammensetzung zur Verwendung nach einem der vorhergehenden Ansprüche, wobei die Zusammensetzung ferner therapeutische Mengen an Cholin, einem Cholinsalz und/oder Cholinester umfasst.

5. Zusammensetzung zur Verwendung nach einem der vorhergehenden Ansprüche, wobei die Zusammensetzung ferner therapeutische Mengen von mindestens einem, vorzugsweise mindestens zwei, am bevorzugtesten allen Antioxidantien umfasst, die aus der Gruppe ausgewählt sind, die aus Selen, Vitamin C und Vitamin E besteht, wobei die Zusammensetzung vorzugsweise mindestens Selen umfasst.

6. Zusammensetzung zur Verwendung nach einem der vorhergehenden Ansprüche, wobei die Summe aus Threonin, Valin, Histidin und Methionin mindestens 12 Gew.%, vorzugsweise mindestens 13 Gew.-%, besonders bevorzugt mindestens 14 Gew.%, besonders bevorzugt 15 - 60 Gew.-%, am bevorzugtesten 16 - 40 Gew.-%, bezogen auf die gesamte proteinhaltige Substanz, beträgt.

7. Zusammensetzung zur Verwendung nach einem der vorhergehenden Ansprüche, wobei die Zusammensetzung Leucin in freier Form umfasst, wobei die Gesamtmenge an L-Leucin, die von der proteinhaltigen Substanz bereitgestellt wird, mindestens 7 Gew.-%, vorzugsweise mindestens 8 Gew.-%, besonders bevorzugt mindestens 9 - 20 Gew.-%, am bevorzugtesten 9 - 15 Gew.-%, bezogen auf die gesamte proteinhaltige Substanz, beträgt.

8. Kombination von therapeutischen Mengen einer mehrfach ungesättigten ω-3-Fettsäure (LCPUFA), die aus der Gruppe ausgewählt ist, die aus Folgendem besteht: Docosahexaensäure (DHA), Eicosapentaensäure (EPA) und Docosapentaensäure (DPA); und mehreren von Uridin, Deoxyuridin, Uridinphosphaten, Uracil oder acyliertem Uridin zur Verwendung bei der therapeutischen Verbesserung, Förderung, Wiederherstellung oder Aufrechterhaltung der Integrität der weißen Substanz bei einem Patienten, der an PKU leidet, und/oder bei einem Patienten, der an Anomalien der weißen Substanz oder einer beeinträchtigten Integrität der weißen Substanz im Zusammenhang mit PKU leidet oder ein erhöhtes Risiko dafür hat, wobei die besagte Kombination mindestens DHA umfasst und ferner therapeutische Mengen an Vitamin D und Vitamin K umfasst, und wobei die Kombination ferner eine proteinhaltige Substanz umfasst, die im Wesentlichen frei von Phenylalanin ist.

9. Zusammensetzung, die für PKU-Patienten geeignet ist, die therapeutische Mengen einer mehrfach ungesättigten ω-3-Fettsäure (LCPUFA) umfasst, die aus der Gruppe ausgewählt ist, die aus Folgendem besteht: Docosahexaensäure (DHA), Eicosapentaensäure (EPA) und Docosapentaensäure (DPA); mehreren von Uridin, Desoxyuridin, Uridinphosphaten, Uracil oder acyliertem Uridin und Vitamin D und Vitamin K, wobei die Zusammensetzung mindestens DHA umfasst und ferner eine proteinhaltige Substanz umfasst, die im Wesentlichen frei von Phenylalanin ist.

10. Zusammensetzung nach Anspruch 9, die ferner therapeutische Mengen von mindestens einem, vorzugsweise mindestens zwei, besonders bevorzugt allen B-Vitaminen umfasst, die aus der Gruppe ausgewählt sind, die aus Vitamin B6, Vitamin B12 und Vitamin B9 besteht.

11. Zusammensetzung nach Anspruch 9 oder 10, wobei die Summe aus Threonin, Valin, Histidin und Methionin mindestens 12 Gew.-%, vorzugsweise mindestens 13 Gew.-%, besonders bevorzugt mindestens 14 Gew.-%, besonders bevorzugt 15 - 60 Gew.-%, am bevorzugtesten 16 - 40 Gew.-%, bezogen auf die gesamte proteinhaltige Substanz, beträgt und/oder wobei die Zusammensetzung Leucin in freier Form umfasst, wobei die Gesamtmenge an L-Leucin, die von der proteinhaltigen Substanz bereitgestellt wird, mindestens 7 Gew.-%, vorzugsweise mindestens 8 Gew.-%, besonders bevorzugt mindestens 9 - 20 Gew.-%, am bevorzugtesten 9 - 15 Gew.-%, bezogen auf die gesamte proteinhaltige Substanz, beträgt.

## Revendications

1. Composition pour utilisation pour améliorer, promouvoir, restaurer ou maintenir de manière thérapeutique l'intégrité de la substance blanche chez un sujet souffrant de PCU et/ou chez un sujet souffrant ou présentant un risque accru d'anomalies de la substance blanche ou d'intégrité altérée de la substance blanche associées à la PCU, ladite composition comprenant des quantités thérapeutiques d'acide gras polyinsaturé ω-3 (LCPUFA) sélectionné parmi le groupe constitué d'acide docosahexaénoïque (DHA), acide eicosapentaénoïque (EPA) et acide docosapentaénoïque (DPA) ; et un ou plusieurs parmi uridine, désoxyuridine, phosphates d'uridine, uracile et uridine acylée, ladite composition comprenant au moins du DHA, ladite composition comprenant en outre des quantités thérapeutiques de vitamine D et de vitamine K, et où la composition comprend en outre une matière protéique qui est essentiellement libre de phénylalanine.

2. Composition pour utilisation selon la revendication 1, où la composition comprend en outre inositol et/ou iode.

3. Composition pour utilisation selon l'une quelconque des revendications précédentes, où la composition comprend des quantités thérapeutiques d'au moins une des vitamines B sélectionnées parmi le groupe consistant en vitamine B6, vitamine B12 et vitamine B9, de préférence B6 et/ou B12.

4. Composition pour utilisation selon l'une quelconque des revendications précédentes, où la composition comprend en outre des quantités thérapeutiques de choline, un sel de choline et/ou un ester de choline.

5. Composition pour utilisation selon l'une quelconque des revendications précédentes, où la composition comprend en outre des quantités thérapeutiques d'au moins un, de préférence au moins deux, de préférence tous les antioxydants sélectionnés parmi le groupe consistant en sélénium, vitamine C et vitamine E, où la composition comprend de préférence au moins sélénium.

6. Composition pour utilisation selon l'une quelconque des revendications précédentes, où la somme de thréonine, valine, histidine et méthionine est au moins 12 % en poids, de préférence au moins 13 % en poids, plus préférablement au moins 14 % en poids, plus préférablement de 15 % à 60 % en poids, plus préférentiellement de 16 à 40 % en poids sur la base de la matière protéique totale.

7. Composition pour utilisation selon l'une quelconque des revendications précédentes, où la composition comprend de la leucine sous forme libre, où la quantité totale de L-leucine fournie par la matière protéique est au moins 7 % en poids, de préférence au moins 8 % en poids, plus préférablement au moins de 9 à 20 % en poids, plus préférentiellement de 9 à 15 % en poids sur la base de la matière protéique totale.

8. Combinaison de quantités thérapeutiques d'acide gras polyinsaturé ω-3 (LCPUFA) sélectionné parmi le groupe consistant en acide docosahexaénoïque (DHA), acide eicosapentaénoïque (EPA) et acide docosapentaénoïque (DPA) ; et un ou plusieurs parmi uridine, désoxyuridine, phosphates d'uridine, uracile et uridine acylée, pour utilisation pour améliorer, favoriser, restaurer ou maintenir de manière thérapeutique l'intégrité de la substance blanche chez un sujet souffrant de PCU et/ou chez un sujet souffrant ou présentant un risque accru d'anomalies de la substance blanche ou d'intégrité altérée de la substance blanche associées à la PCU, ladite combinaison comprenant au moins du DHA, et comprenant en outre des quantités thérapeutiques de vitamine D et de vitamine K, et où la combinaison comprend en outre une matière protéique qui est essentiellement libre de phénylalanine.

9. Composition adaptée aux patients atteints de PCU, comprenant des quantités thérapeutiques d'acide gras polyinsaturé ω-3 (LCPUFA) sélectionné parmi le groupe consistant en acide docosahexaénoïque (DHA), acide eicosapentaénoïque (EPA) et acide docosapentaénoïque (DPA) ; un ou plusieurs parmi uridine, désoxyuridine, phosphates d'uridine, uracile et uridine acylée, et vitamine D et vitamine K, où la composition comprend au moins du DHA et comprend en outre une matière protéique qui est essentiellement libre de phénylalanine.

10. Composition selon la revendication 9, comprenant en outre des quantités thérapeutiques d'au moins une, de préférence au moins deux, plus préférablement toutes les vitamines B sélectionnées parmi le groupe consistant en vitamine B6, vitamine B12 et vitamine B9.

11. Composition selon la revendication 9 ou 10, où la somme de thréonine, valine, histidine et méthionine est au moins 12 % en poids, de préférence au moins 13 % en poids, plus préférablement au moins 14 % en poids, plus préférablement de 15 à 60 % en poids, plus préférentiellement de 16 à 40 % en poids sur la base de la matière protéique totale et/ou où la composition comprend de la leucine sous forme libre, où la quantité totale de L-leucine fournie par la matière protéique est au moins 7 % en poids, de préférence au moins 8 % en poids, plus préférablement au moins de 9 à 20 % en poids, plus préférentiellement de 9 à 15 % en poids, sur la base de la matière protéique totale.
